(19) Europäisches Patentamt
European Patent Office
Office européen des brevets

(11) **EP 0 940 120 B1**

(12) **FASCICULE DE BREVET EUROPEEN**

(45) Date de publication et mention
de la délivrance du brevet:
**28.04.2004 Bulletin 2004/18**

(51) Int Cl.⁷: **A61B 5/05**, G01G 19/44

(21) Numéro de dépôt: **99400462.0**

(22) Date de dépôt: **25.02.1999**

(54) **Appareil et procédé de mésure de la composition corporelle**

Vorrichtung und Verfahren zum Messen der Zusammensetzung eines Körpers

Apparatus and method for measuring the composition of a body

(84) Etats contractants désignés:
**BE CH DE ES GB IT LI NL**

(30) Priorité: **03.03.1998 FR 9802550**

(43) Date de publication de la demande:
**08.09.1999 Bulletin 1999/36**

(73) Titulaire: **SEB S.A.**
**69130 Ecully (FR)**

(72) Inventeurs:
• **Sarrazin, Michel**
**74150 Massingy (FR)**
• **Duborper, Alain**
**74150 Sales (FR)**

(74) Mandataire: **Keib, Gérard et al**
**Novagraaf Technologies,**
**122, rue Edouard Vaillant**
**92593 Levallois Perret Cedex (FR)**

(56) Documents cités:
**WO-A-83/03746**        **WO-A-96/08198**
**WO-A-97/00642**        **DE-U- 9 005 415**
**FR-A- 2 698 779**

## Description

La présente invention concerne un appareil et un procédé de mesure de la composition corporelle d'un individu, ledit appareil comportant un premier module électronique destiné à mesurer l'impédance bioélectrique du corps et comprenant une source de courant destinée à délivrer un signal électrique variable qui traverse le corps de l'individu lorsque ce dernier est relié à l'appareil de mesure.

La mesure de la composition corporelle est utilisée pour apprécier le besoin nutritionnel d'un individu et est basée, de façon connue en soi, sur la mesure de l'impédance bioélectrique du corps de cet individu. Elle fournit des informations sur le rapport entre la masse maigre et la masse grasse ainsi que sur la quantité d'eau contenues dans le corps.

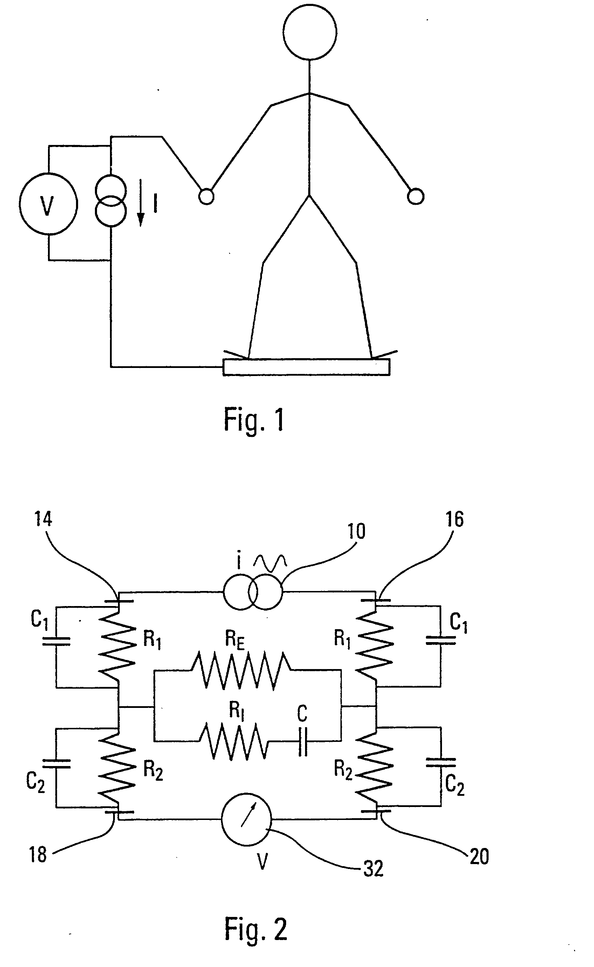

Les méthodes et les appareils de mesure de l'impédance bioélectrique connus sont basés sur un principe consistant à appliquer un courant alternatif faible (50 à 1500 µA) au corps de l'individu au moyen de deux ou de quatre électrodes comme cela est schématisé à la figure 1. La différence de potentiel qui apparaît entre celles-ci permet de calculer l'impédance corporelle globale Z du corps, ce dernier étant assimilé à une suspension de cellules dans une solution électrolytique. La détermination de l'impédance corporelle globale Z est effectuée à partir d'un modèle électrique du corps, tel que le modèle de Fricke illustré à la figure 2, en tenant compte de la fréquence $f_c$ du courant utilisé, de la résistance opposée au passage du courant électrique par l'eau et par les électrolytes intra et extra cellulaires. Les résistances desdits électrolytes sont représentées respectivement par $R_I$ et par $R_E$ sur le modèle de Fricke. Le calcul de l'impédance bioélectrique tient compte également de la réactance $X_{fc}$ de la membrane du corps qui a pour effet de provoquer un déphasage Θ entre le courant appliqué et la tension mesurée. En référence à la figure 2, $R_1$, $R_2$ $C_1$, $C_2$ représentent respectivement les résistances et les capacités de contact des électrodes. Les équations qui lient ces facteurs sont les suivantes :

$$Z^2 = R^2 + X_{fc}^2$$

$$X_{fc} = |Z| \times \sin\Theta$$

$$R = |Z| \times \cos\Theta$$

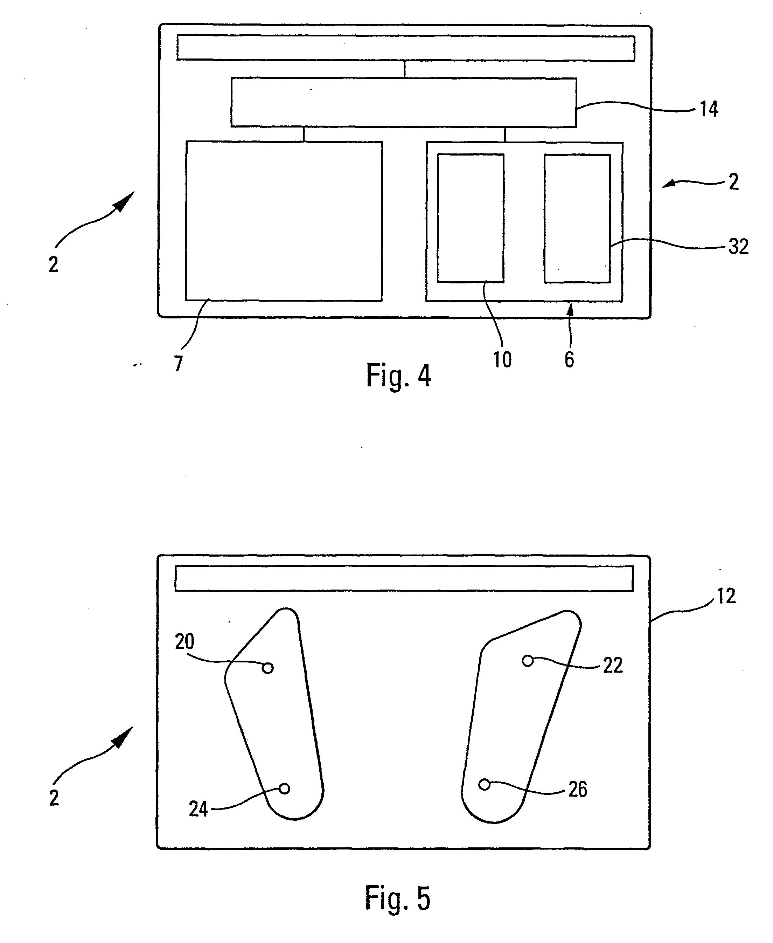

Le lieu d'impédance de ces équations est un demi-cercle centré sur l'axe des abscisses comme cela est illustré par la figure 3.

Un inconvénient des appareils basés sur cette méthode provient du fait que pour déterminer les valeurs de l'impédance du corps à partir du diagramme de la figure 3, il est nécessaire d'utiliser plusieurs fréquences d'excitation. En outre, il s'est avéré qu'une fréquence trop basse ne permet pas d'explorer correctement le secteur intracellulaire car la capacité membranaire présente une réactance trop élevée à cette fréquence. Par ailleurs, ces appareils nécessitent la mesure du déphasage Θ qui est indispensable pour, en plus de la mesure de l'impédance Z, estimer la partie résistive R qui donne la meilleure précision pour le calcul de la masse maigre et de l'eau totale.

On connaît des appareils utilisant quatre électrodes et une fréquence unique de 50 KHz pour laquelle le déphasage est maximal. Ces appareils ne permettent pas de mesurer spécifiquement l'impédance intra-cellulaire $R_I$ et l'impédance extra-cellulaire $R_E$ du corps. Ils fournissent seulement une mesure globale de l'impédance du corps.

Le but de la présente invention est de pallier les inconvénients décrits ci-dessus.

Selon l'invention, le signal électrique délivré par la source de courant présente une forme d'onde carrée dont la durée est réglable de manière à déterminer directement l'impédance corporelle globale, l'impédance intracellulaire et l'impédance extra-cellulaire du corps.

Le réglage de la durée du signal carré permet de mesurer la résistance équivalente du modèle de Ficke, sans recourir à plusieurs fréquences. En outre, l'effet capacitif des membranes cellulaires peut être déterminé directement à partir de la mesure desdites impédances intra-cellulaire et extra-cellulaire.

Avantageusement, afin d'adapter la mesure à des individus physiologiquement différents, la fréquence du signal électrique est choisie entre 2 KHz et 400 KHz.

D'autres caractéristiques et avantages de l'invention ressortiront de la description qui va suivre, prise à titre d'exemple non limitatif, en référence aux figures annexées dans lesquelles:

- la figure 1 représente un schéma de principe connu de mesure de l'impédance bioélectrique basé sur l'utilisation de deux électrodes;
- la figure 2 représente schématiquement un dispositif de mesure à quatre électrodes selon l'art antérieur;
- la figure 3 représente un diagramme d'impédance utilisé dans les méthodes de l'art antérieur pour calculer l'impédance bioélectrique du corps humain;
- la figure 4 représente un schéma bloc d'un appareil de mesure de la quantité de matière grasse dans un corps selon l'invention ;
- la figure 5 représente schématiquement une vue de dessus de l'appareil de la figure 4 ;
- la figure 6 représente un schéma illustrant le modèle bioélectrique du corps humain ;
- la figure 7 représente un schéma illustrant la forme d'onde d'un courant électrique délivré par une source équipant un appareil selon l'invention ;

- la figure 8 illustre schématiquement une variante du courant utilisé dans un appareil selon l'invention.

La figure 4 représente schématiquement un appareil 2 de mesure de la composition corporelle d'un individu, qui peut être un être humain ou un animal, ledit appareil comportant un premier module électronique 6 destiné à mesurer l'impédance bioélectrique du corps et comprenant une source de courant 10 destinée à délivrer un signal électrique variable 11 qui traverse le corps de l'individu lorsque ce dernier est relié à l'appareil 2.

Ledit appareil 2 comporte également un deuxième module électronique 7 destiné à mesurer le poids de l'individu. Le premier module 6 et le deuxième module 7 sont reliés à une unité de calcul 14 qui peut être un microprocesseur ou un microcontrôleur adapté au traitement à effectuer. Ladite unité de calcul 14 reçoit les valeurs respectives du poids et de l'impédance bioélectrique mesurés et calcule la quantité de masse grasse et de masse maigre contenues dans le corps de l'individu, en fonction de ces valeurs et à partir de formules connues en soi dont un développement complet est décrit dans le brevet français n° 2 698 779. Par ailleurs, l'unité de calcul 14 est reliée à un moyen d'affichage 15 qui permet de visualiser simultanément ou séquentiellement lesdites valeurs mesurées et lesdites valeurs calculées afin de suivre en temps réel l'évolution des grandeurs mesurées.

Comme on peut le voir sur la figure 5, l'appareil 2 comporte deux électrodes d'excitation 20, 22 destinées à appliquer le signal électrique 11 entre un premier point et un deuxième point situés sur le corps de l'individu, et deux électrodes de mesure 24 et 26 aux bornes desquelles est mesurée une tension électrique 30. Les électrodes 24 et 26 sont reliées à une unité de mesure 32 intégrée au deuxième module électronique 7.

Dans l'appareil de l'invention le signal électrique 11 délivré par la source de courant 10 présente une forme d'onde carrée dont la durée peut être réglée par l'unité de traitement 14 , en fonction des mesures et des traitements à effectuer.

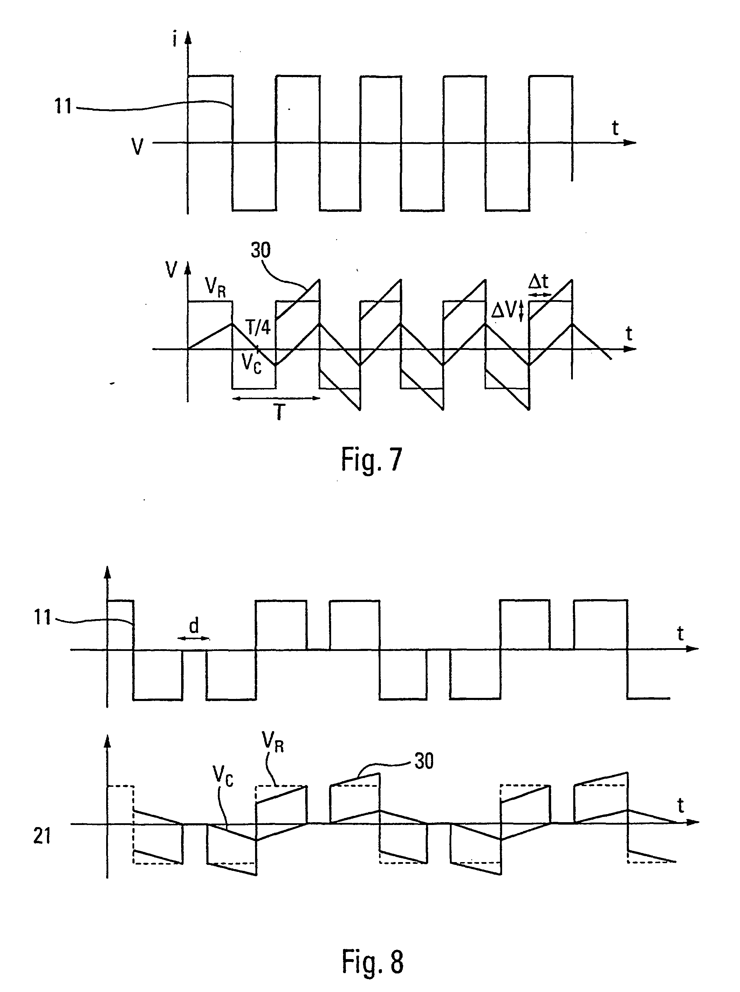

Ainsi comme on peut le voir à la figure 8, le signal électrique 11 peut être annulé pendant une durée prédéterminée d de manière à annuler parfaitement l'effet capacitif des membranes cellulaires. Ledit signal électrique 11 peut également être maintenu à une valeur constante pendant une durée préalablement déterminée par l'utilisateur. Ladite durée peut être fixée par programmation de l'unité de calcul 14 ou par un moyen de réglage extérieur que l'utilisateur peut manipuler pendant la mesure.

Comme on peut le voir à la figure 7, la partie capacitive du dipôle représentant le corps se charge avec une constante de temps $R_I.C$, tandis que la partie résistive suit parfaitement la rampe du signal 11. La droite de charge $V_c$ du condensateur C coupe périodiquement le palier de la partie résistive. En effectuant la mesure à cet instant précis, il est possible de calculer la résistance R équivalente aux résistance $R_E$ et $R_I$ en parallèle.

Cette résistance $R_E$ est déterminée par la relation $R_E = \frac{V_B - V_c}{i}$, les tensions $V_b$ et $V_c$ étant mesurées à un instant correspondant à la saturation du condensateur C. La résistance $R_I$ est déduite de la relation $R = \frac{R_E \times R_I}{R_E + R_I}$, et la capacité C est calculée à partir de la relation $\Delta V = \frac{i}{C} \times \Delta t$ dans laquelle $\Delta V$ représente une variation de la tension $V_c$ pendant un intervalle $\Delta t$.

Le procédé de mesure consiste donc à appliquer le signal carré 11 entre les électrodes d'excitation 20 et 22, et à mesurer la tension 30 entre les électrodes de mesure 24 et 26 à des instant pendant lesquels l'effet capacitif du tissus du corps de l'individu est minimal voire nul. A cet effet, l'unité de mesure 32 est programmée de manière à saisir les valeurs mesurées de la tension 30 à chaque passage par zéro de la tension $V_c$. Cette mesure est effectuée par rapport à une tension de référence stable obtenue aux bornes d'une résistance de précision $R_r$ .

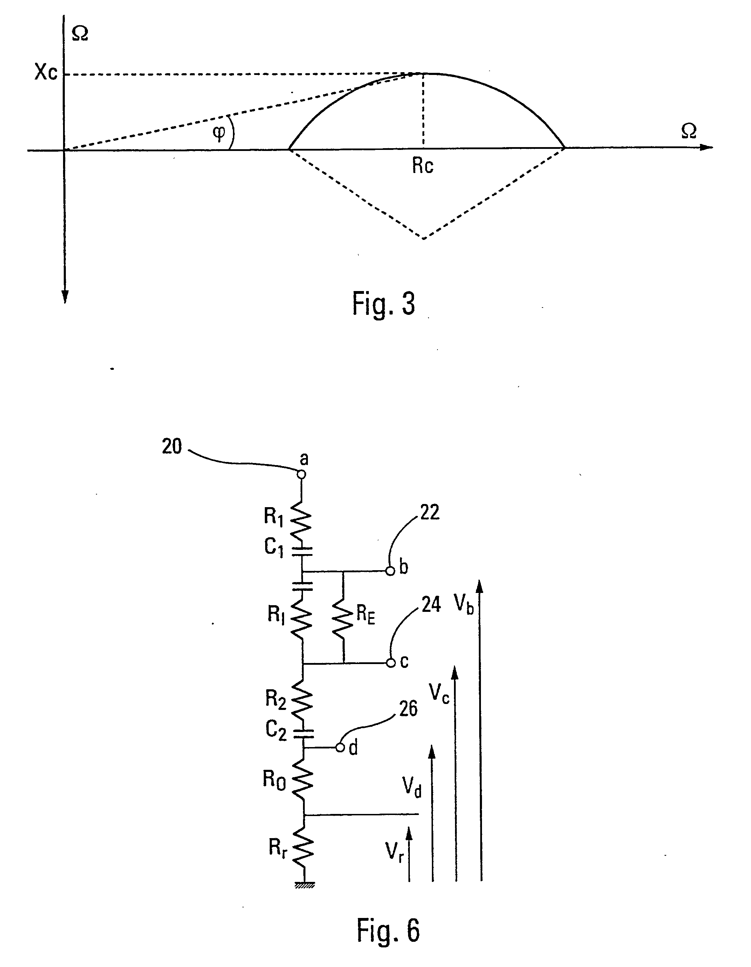

Le principe de cette mesure est illustré par la figure 6, sur laquelle sont représentés les différents points de connexion des électrodes 20, 22, 24 et 26. Comme on peut le voir sur cette figure, une tension $V_d$ est mesurée à la borne 26, une tension $V_c$ est mesurée à la borne 24 et une tension $V_b$ est mesurée à la borne 22. Les valeurs mesurées ainsi que le poids de l'individu sont ensuite transmis à l'unité de calcul 14 qui détermine, en temps réel, la quantité de matière grasse, et la quantité de matière maigre ainsi que la quantité d'eau contenues dans le corps de l'individu.

Selon un deuxième mode de réalisation de l'invention, on annule le signal de courant 11 au milieu de chaque alternance, comme cela est illustré à la figure 8, de manière à annuler parfaitement la tension $V_c$. Ceci permet d'améliorer la stabilité de la tension mesurée 30.

Le premier module 6 peut être intégré dans un pèse-personne classique. Il peut également être utilisé pour suivre l'évolution d'un traitement destiné à apprécier le besoin nutritionnel d'un individu. L'appareil selon l'invention peut également servir d'indicateur de l'évolution de la composition corporelle locale d'un individu au cours d'une séance de traitement de la cellulite ou au cours de séances de massage.

## Revendications

1. Appareil (2) de mesure de la composition corporelle d'un individu comportant un premier module électronique (6) destiné à mesurer une impédance bioélectrique et comprenant au moins une source de courant (10) destinée à délivrer un signal électrique variable (11) qui traverse le corps de l'individu lorsque ce dernier est relié à l'appareil de mesure (2), **caractérisé en ce que** ledit signal électrique (11) présente une forme d'onde carrée dont la durée est réglable de manière à déterminer directement l'impédance corporelle globale, l'impédance intracellulaire et l'impédance extra-cellulaire du corps de l'individu.

2. Appareil selon la revendication 1, **caractérisé en ce qu'**il comporte en outre un deuxième module électronique (7) destiné à mesurer le poids de l'individu.

3. Appareil selon la revendication 2, **caractérisé en ce que** le premier module (6) et le deuxième module (7) sont reliés à une unité de calcul (14) apte à calculer, en fonction du poids et de l'impédance bioélectrique mesurés, la masse grasse, la masse maigre et la quantité d'eau contenues dans le corps de l'individu.

4. Appareil selon la revendication 1, **caractérisé en ce que** la fréquence dudit signal électrique (11) est choisie entre 2 KHz et 400 KHz.

5. Appareil selon la revendication 3, **caractérisé en ce que** l'unité de calcul (14) est un microprocesseur adapté au traitement à effectuer.

6. Appareil selon l'une des revendications 1 à 5, **caractérisé en ce qu'**il comporte un moyen d'affichage (15) des valeurs mesurées et des valeurs calculées.

7. Appareil selon l'une des revendications 1 à 6, **caractérisé en ce qu'**il comporte au moins deux électrodes d'excitation (20 ; 22) destinées à appliquer le signal électrique (11) entre un premier point et un deuxième point sur le corps de l'individu et au moins deux électrodes de mesure (24 ;26) aux bornes desquelles est mesurée une tension électrique (30).

8. Pèse-personne comportant un premier module électronique (6) destiné à mesurer une impédance bioélectrique et comprenant au moins une source de courant (10) destinée à délivrer un signal électrique variable (11) qui traverse le corps d'un individu, **caractérisé en ce que** ledit signal électrique (11) présente une forme d'onde carrée dont la durée est réglable de manière à déterminer directement l'impédance intra-cellulaire et l'impédance extra-cellulaire du corps de l'individu.

9. Procédé de mesure de l'impédance bioélectrique du corps d'un individu, **caractérisé en ce que** l'on applique un signal carré (11) de durée réglable entre deux électrodes d'excitation (20) (22) reliées à l'individu et l'on mesure une tension (30) entre deux électrodes de mesure (24) et (26), reliées également à l'individu, la mesure de ladite tension (30) étant effectuée à des instants pendant lesquels l'effet capacitif du tissu du corps de l'individu est nul.

10. Procédé selon le revendication 9, **caractérisé en ce que** la durée du signal électrique peut être réglée par l'unité de calcul (14), en fonction des mesures et des traitements à effectuer.

## Claims

1. A device (2) for measuring the composition of the body of a person including a first electronic module (6) adapted to measure a bioelectrical impedance and comprising at least one current source (10) adapted to deliver a variable electrical signal (11) that passes through said body of said person when said person is connected to said measuring device (2), **characterized in that** said electrical signal (11) is a squarewave the duration of which is adjustable to determine the global body impedance, the intracellular impedance and the extracellular impedance of said body of said person directly.

2. The device according to claim 1, **characterized in that** it further includes a second electronic module (7) for measuring the weight of said person.

3. The device according to claim 2, **characterized in that** said first module (6) and said second module (7) are connected to a calculator unit (14) adapted to calculate from the measured weight and the measured bioelectrical impedance, the fat body mass, the lean body mass and the amount of water contained in said body of said person.

4. The device according to claim 1, **characterized in that** the frequency of said electrical signal (11) is chosen in the range 2 kHz to 400 kHz.

5. The device according to claim 3, **characterized in that** said calculator unit (14) is a microprocessor adapted to carry out the necessary processing.

6. The device according to anyone of the claims 1 to 5, **characterized in that** it includes displaying means (15) for measured values and calculated values.

7. The device according to anyone of the claims 1 to 6, **characterized in that** the device includes at least two excitation electrodes (20 ; 22) adapted to apply said electrical signal (11) between first and second points on said body of said person and at least two measuring electrodes (24 ; 26) between which an electrical voltage (30) is measured.

8. Scales including a first electronic module (6) for measuring a bioelectrical impedance and comprising at least one current source (10) adapted to deliver a variable electrical signal (11) that passes through the body of a person, **characterized in that** said electrical signal (11) is a squarewave the duration of which is ajustable to determine the intracellular impedance and the extracellular impedance of said body of said person directly.

9. A method of measuring the bioelectrical impedance of the body of a person, **characterized in that** a squarewave signal (11) of adjustable duration is applied between two excitation electrodes (20, 22) connected to said person, and that a voltage (30) is measured between two measuring electrodes (24, 26) also connected to said person, said voltage (30) being measured at times during which the tissue of said body of said person has no capacitive effect.

10. The method claimed in claim 9 **characterized in that** said duration of said electrical signal can be varied by the calculator unit (14) to suit the measurements and the processing to be effected.

**Patentansprüche**

1. Vorrichtung (2) zur Messung der körperlichen Zusammensetzung eines Individuums, mit einem ersten elektronischen Modul (6), das zur Messung einer bioelektrischen Impedanz bestimmt ist und zumindest eine Stromquelle (10) aufweist, die dazu bestimmt ist, ein variables elektrisches Signal (11) abzugeben, welches durch den Körper des Individuums läuft, wenn das Individuum an die Vorrichtung zur Messung (2) angeschlossen ist, **dadurch gekennzeichnet, dass** das elektrische Signal (11) einen Rechteckwellenverlauf hat, dessen Dauer so regelbar ist, dass direkt die gesamte körperliche Impedanz, die intrazelluläre Impedanz und die extrazelluläre Impedanz des Körpers des Individuums bestimmt werden können.

2. Vorrichtung nach Anspruch 1, **dadurch gekennzeichnet, dass** sie zudem ein zweites elektronisches Modul (7) aufweist, das zur Messung des Gewichts des Individuums bestimmt ist.

3. Vorrichtung nach Anspruch 2, **dadurch gekennzeichnet, dass** das erste Modul (6) und das zweite Modul (7) an eine Recheneinheit (14) angeschlossen sind, die angepasst ist, um in Abhängigkeit vom gemessenen Gewicht und der gemessenen bioelektrischen Impedanz die Fettmasse, die Magermasse und die in dem Körper des Individuums enthaltene Wassermenge zu berechnen.

4. Vorrichtung nach Anspruch 1, **dadurch gekennzeichnet, dass** die Frequenz des elektrischen Signals (11) derart gewählt ist, dass sie zwischen 2 KHz und 400 KHz liegt.

5. Vorrichtung nach Anspruch 3, **dadurch gekennzeichnet, dass** die Recheneinheit (14) ein Mikroprozessor ist, der an die durchzuführende Verarbeitung angepasst ist.

6. Vorrichtung nach einem der Ansprüche 1 bis 5, **dadurch gekennzeichnet, dass** sie eine Anzeigeeinrichtung (15) für die gemessenen Werte und die berechneten Werte aufweist.

7. Vorrichtung nach einem der Ansprüche 1 bis 6, **dadurch gekennzeichnet, dass** sie zumindest zwei Anregungselektroden (20; 22) aufweist, die dazu bestimmt sind, das elektrische Signal (11) zwischen einem ersten Punkt und einem zweiten Punkt an den Körper des Individuums anzulegen und zumindest zwei Messelektroden (24; 26) aufweist, an deren Anschlussklemmen eine elektrische Spannung (30) gemessen wird.

8. Personenwaage mit einem ersten elektronischen Modul (6), das dazu bestimmt ist, eine bioelektrische Impedanz zu messen und zumindest eine Stromquelle (10) aufweist, die dazu bestimmt ist, ein variables elektrisches Signal (11) abzugeben, welches den Körper eines Individuums durchläuft, **dadurch gekennzeichnet, dass** das elektrische Signal (11) einen Rechteckwellenverlauf hat, dessen Dauer so regelbar ist, dass direkt die intrazelluläre Impedanz und die extrazelluläre Impedanz des Körpers des Individuums bestimmt werden können.

9. Verfahren zur Messung der bioelektrischen Impedanz des Körpers eines Individuums, **dadurch gekennzeichnet, dass** ein Rechtecksignal regelbarer Spannung zwischen zwei Anregungselektroden (20) (22) angelegt wird, die an das Individuum angeschlossen sind, und dass eine Spannung (30) zwischen zwei Messelektroden (24) und (26) gemessen wird, die ebenfalls an das Individuum angeschlossen sind, wobei die Messung der Spannung (30) zu Zeitpunkten ausgeführt wird, während denen der kapazitive Effekt des Gewebes des Körpers des Individuums Null ist.

10. Verfahren nach Anspruch 9, **dadurch gekennzeichnet, dass** die Dauer des elektrischen Signals durch die Recheneinheit (14) abhängig von den durchzuführenden Messungen und Verarbeitungen geregelt werden kann.

Fig. 1

Fig. 2

Fig. 4

Fig. 5

Fig. 3

Fig. 6

Fig. 7

Fig. 8